(19) Europäisches Patentamt
European Patent Office
Office européen des brevets

(11) EP 3 070 074 A1

(12) **EUROPEAN PATENT APPLICATION**

(43) Date of publication:
**21.09.2016 Bulletin 2016/38**

(51) Int Cl.:
***C07C 1/24*** *(2006.01)* ***C07C 11/04*** *(2006.01)*

(21) Application number: **15160076.4**

(22) Date of filing: **20.03.2015**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(71) Applicant: **Rhodia Operations**
**75009 Paris (FR)**

(72) Inventor: **Ohtake, Naotaka**
**Anan City, 774-0022 (JP)**

(74) Representative: **Vande Gucht, Anne et al**
**Solvay S.A.**
**Intellectual Assets Management**
**Rue de Ransbeek, 310**
**1120 Bruxelles (BE)**

(54) **Dehydration of alcohols**

(57) The present invention concerns a method for producing an alkene, the method comprising a step of converting an alcohol by a dehydration step into an alkene with cerium oxide particles as catalyst. Method of the present invention notably permits the production of ethanol, propanol, butanol and pentanol.

EP 3 070 074 A1

## Description

The present invention concerns a method for producing an alkene, the method comprising a step of converting an alcohol by a dehydration step into an alkene with cerium oxide particles as catalyst. Method of the present invention notably permits the production of ethanol, propanol, butanol and pentanol.

## PRIOR ART

The following discussion of the prior art is provided to place the invention in an appropriate technical context and enable the advantages of it to be more fully understood. It should be appreciated, however, that any discussion of the prior art throughout the specification should not be considered as an express or implied admission that such prior art is widely known or forms part of common general knowledge in the field.

A large number of chemical compounds are currently derived from petrochemicals. Alkene, such as ethylene, propylene, the different butylenes, or else the pentylenes, are used in the plastics industry, for example for producing polypropylene or polyethylene, and in other areas of the chemical industry and that of fuels. Ethylene, the simplest alkene, lies at the heart of industrial organic chemistry : it is the most widely produced organic compound in the world. It is used in particular to produce polyethylene, a major plastic. Ethylene can also be converted to many industrially useful products by reaction (e.g. by oxidation or halogenation). Propylene plays a similarly important role : its polymerization results in a plastic material, polypropylene. The technical properties of this product in terms of resistance, density, solidity, deformability, and transparency are unequalled. Butylene exists in four forms, one of which, isobutylene, enters into the composition of methyl-tert-butyl-ether (MTBE), an anti-knock additive for automobile fuel. Isobutylene can also be used to produce isooctene, which in turn can be reduced to isooctane (2,2,4-trimethylpentane); the very high octane rating of isooctane makes it the best fuel for so-called "gasoline" engines. Amylene, hexene and heptene exist in many forms according to the position and configuration of the double bond. These products have real industrial applications but are less important than ethylene, propylene or butylenes. All these alkenes are currently produced by catalytic cracking of petroleum products, or by a derivative of the Fischer-Tropsch process in the case of hexene, from coal or gas. Their production costs are therefore tightly linked to the price of oil. Moreover, catalytic cracking is sometimes associated with considerable technical difficulties which increase process complexity and production costs.

Dehydration of alcohols using aluminium oxide as catalyst is a simple way of making gaseous alkenes, like ethane (ethylene). However, aluminium oxide catalysts leads to insufficient results with respect to both alcohol conversion and alkene yield.

Dehydration of alcohols using an acid catalyst is also known. The acid catalysts normally used are either concentrated sulphuric acid or concentrated phosphoric(V) acid, $H_3PO_4$. However, $HSO_4^-$ or $H_2PO_4^-$ anions, formed when $H_2SO_4$ or $H_3PO_4$ protonate alcohols, are not good nucleophiles and do not to displace $OH_2^+$ groups. Either $HSO_4^-$ or $H_2PO_4^-$ can combine with intermediate carbocations that may form in E1 reactions, but the resultant products are unstable and either undergo elimination reactions to form alkenes, or revert to alcohols by substitution. Moreover, concentrated sulphuric acid produces difficult results as it oxidises some of the alcohol to carbon dioxide and is reducing itself to sulphur dioxide. Both of these gases have to be then removed from the alkene. Furthermore, mineral acids (HCl, HBr, or HI) give substitution products (iodoalkanes, bromoalkanes, or chloroalkanes) rather than alkenes.

There is still a need then for efficient and environmentally friendly methods of producing alkenes.

## INVENTION

The present invention concerns a method for producing an alkene comprising a step of converting an alcohol by a dehydration step into an alkene with cerium oxide particles as catalyst, wherein said cerium oxide particles have the following properties :

- a specific surface area (SBET) comprised between 100 and 300 $m^2/g$, after calcination at 400°C for 10 hours; and
- a weight loss comprised between -1.0 and +1.0 %, between a temperature of 350°C and 1000°C, as measured by a Thermal Gravimetric Analysis (TGA).

It is therefore an object of the present invention to provide a method for producing an alkene by dehydration of alcohol with higher conversion and yield in comparison with the catalysts of the prior art having not the above identified drawbacks. Indeed, catalytic performances of cerium oxide particles occur without any high cost and non-environmental friendly metals, such as Pd, Lu, Ni or Ag, or strong acid. Moreover, method of the present invention permit to obtain alkenes of interest with a high selectivity in comparison with catalysts of the prior art.

Other characteristics, details and advantages of the invention will emerge even more fully upon reading the description which follows.

## DEFINITIONS

Throughout the description, including the claims, the term "comprising one" should be understood as being synonymous with the term "comprising at least one", unless otherwise specified, and "between" should be understood as being inclusive of the limits.

It is specified that, in the continuation of the description, unless otherwise indicated, the values at the limits are included in the ranges of values which are given.

The contents are given as oxides, unless otherwise indicated. The cerium oxide is in the form of cerium oxide ($CeO_2$).

In the continuation of the description, the term "specific surface area" is understood to mean the BET specific surface area determined by nitrogen adsorption in accordance with standard ASTM D 3663-78 laid down from the Brunauer-Emmett-Teller method described in the periodical "The Journal of the American Chemical Society, 60, 309 (1938)".

As used herein and unless defined otherwise, the term "hydrocarbyl" refers to a monovalent hydrocarbon group, i.e. a group consisting of carbon atoms and hydrogen atoms, which group is connected to the remainder of the compound of formula (I) via a carbon-to-carbon single bond and may be saturated or unsaturated, linear, branched or cyclic, aliphatic or aromatic. A "$C_{1-11}$ hydrocarbyl" denotes a hydrocarbyl having 1 to 11 carbon atoms.

As used herein and unless defined otherwise, the term "alkyl" refers to a monovalent saturated aliphatic (i.e. non-aromatic) acyclic hydrocarbon group which may be linear or branched and does not comprise any carbon-to-carbon double bond or any carbon-to-carbon triple bond.

As used herein and unless defined otherwise, the term "alkenyl" refers to a monovalent unsaturated aliphatic acyclic hydrocarbon group which may be linear or branched and comprises at least one carbon-to-carbon double bond while it does not comprise any carbon-to-carbon triple bond.

As used herein and unless defined otherwise, the term "alkynyl" refers to a monovalent unsaturated aliphatic acyclic hydrocarbon group which may be linear or branched and comprises at least one carbon-to-carbon triple bond and optionally one or more carbon-to-carbon double bonds.

As used herein and unless defined otherwise, the term "cycloalkyl" refers to a monovalent cyclic saturated aliphatic hydrocarbon group which does not comprise any carbon-to-carbon double bond or any carbon-to-carbon triple bond. Non-limiting examples of cycloalkyl groups are cyclopropyl, cyclobutyl, cyclopentyl or cyclohexyl.

As used herein and unless defined otherwise, the term "cycloalkenyl" refers to a monovalent cyclic unsaturated aliphatic hydrocarbon group which comprises at least one carbon-to-carbon double bond and does not comprise any carbon-to-carbon triple bond. Non-limiting examples of cycloalkyl groups are cyclopropenyl, cyclobutenyl, cyclopentenyl, cyclohexenyl or cyclohexadienyl.

As used herein and unless defined otherwise, the term "aryl" refers to a monovalent aromatic hydrocarbon group, including bridged ring and/or fused ring systems, containing at least one aromatic ring. "Aryl" may, for example, refer to phenyl, naphthyl or anthracenyl.

## DETAILS OF THE INVENTION

The cerium oxide particles according to the present invention comprise at least a cerium oxide.

The cerium oxide particles have a specific surface area (SBET) comprised between 100 and 300 $m^2/g$, after calcination at 400°C for 10 hours, preferably comprised between 120 and 300 $m^2/g$. Preferably, cerium oxide particles may have a specific surface area (SBET) comprised between 30 and 65 $m^2/g$, after calcination at 900°C for 5 hours, preferably comprised between 40 and 65 $m^2/g$.

The specific surface area referred to in the present specification is measured according to the BET method utilizing absorption of nitrogen gas, which is the most standard method for measuring the specific surface area of powders.

Total pore volume of cerium oxide particles may be comprised between 0.7 and 1.5 ml/g after calcination at 900°C for 5 hours, under air; preferably comprised between 1.0 and 1.5 ml/g. The total pore volume may be measured by ordinary mercury porosimeter.

Cerium oxide particles may have a S1/S2 ratio comprised between 0.45 and 0.7 taken after calcination at 800°C for 2 hours. Cerium oxide particles may have a S1/S2 ratio comprised between 0.25 and 0.5 taken after calcination at 900°C for 5 hours.

Said S1/S2 ratio is a ratio of the area (S1) defined by a baseline and a TPR curve in a temperature range of 200 to 600°C to the area (S2) defined by said baseline and said TPR curve in a temperature range of 600 to 1000°C. A higher S1/S2 ratio of a cerium oxide is expected to result in a higher oxygen absorbing and desorbing capability and higher activity to purify exhaust gas at a lower temperature. As used herein, the "baseline" means a line segment drawn from the point on the TPR curve corresponding to 200°C in a parallel to the axis representing temperature, up to 1000°C. The TPR may be performed as described in U.S. Pat No. 7,361,322.

Cerium oxide particles may also comprise at least one metallic element oxide, other than cerium oxide, for instance in a proportion comprised between 1 and 40 % by weight of oxide, preferably in a proportion comprised between 1 and 20 % by weight of oxide. Oxide refers there to final mixed oxide defined as integration of cerium oxide and metallic element oxide.
Metallic element of metallic element oxide is preferably chosen in the group consisting of:

- transition metal elements, such as Zr,
- poor metal elements, such as Al,
- rare earth elements, such as La, Pr, Nd and Y, and
- alkaline earth metal elements, such as Sr.

Metallic element oxides are preferably chosen in the group consisting of: lanthanum oxide ($La_2O_3$), praseodymium oxide ($Pr_6O_{11}$), neodymium oxide ($Nd_2O_3$) and yttrium oxide ($Y_2O_3$).
Cerium oxide particles as described above or as obtained by means of the preparation process previously described may be in the form of powders, but they can optionally be formed so as to be in the form of granules, pellets, foams, beads, cylinders or honeycombs of variable dimensions.
Cerium oxide particles of the present invention provide a weight loss comprised between -0.6 and +0.5 %, between a temperature of 350°C and 1000°C, preferably comprised between -0.5 and +0.2 %.
The weight loss may be measured by Thermal Gravimetric Analysis (TGA) in which changes in physical and chemical properties of materials are measured as a function of increasing temperature with constant heating rate. The sample may be heated from ambient temperature to 1000°C in the air with heating rate of 10°C/min. The weight loss of the samples is calculated by the following calculation.

$$\text{Weight loss (\%)} = (A-B)/A*100$$

A : weight of the sample at 350°C detected by TG
B : weight of the sample at 1000°C detected by TG

Cerium oxide particles may be produced according to the method described in U.S. Pat No. 7,361,322.
Cerium oxide particles may notably be obtained by a method comprising at least the steps of:

(a) providing a cerium solution not less than 90 mol % of which cerium ions are tetravalent,
(b) holding said cerium solution prepared in step (a) at a temperature form 60 to 220°C under heating,
(c) cooling said heated cerium solution,
(d) adding a precipitant to said cooled cerium solution to obtain a precipitate, and
(e) calcining said precipitate.

According to the present method, first a cerium solution not less than 90 mol % of which cerium ions are tetravalent is provided in step (a). In step (a), the cerium solution not less than 90 mol % of which cerium ions are tetravalent, may preferably be a ceric nitrate solution. A ceric nitrate solution initially contains 250 g/L of cerium in terms of cerium oxide, and has an initial acid concentration of usually 0.1 to 1 N. The initial acid concentration relates to the acid concentration in the subsequent reaction. If the acid concentration is too low, the crystallinity of the precipitate to be discussed later may not be improved sufficiently, resulting in low heat resistance of the objective ceric oxide. If the acid concentration is too high, excess base is required in the neutralization reaction for precipitating cerium, thus being industrially disadvantageous.
Thus the acid concentration of the cerium solution may be adjusted to usually 5 to 150 g/L, preferably 10 to 120 g/L, more preferably 15 to 100 g/L, in terms of cerium oxide, usually with water, preferably with deionized water.
According to the present method, next the cerium solution prepared in step (a) is held at 60 to 220°C under heating to cause reaction of the cerium solution in step (b). Any reaction vessel may be used in step (b) without critical limitation, and either a sealed vessel or an open vessel may be used. Specifically, an autoclave reactor may preferably be used.
In step (b), the temperature for holding under heating is 60 to 220°C, preferably 80 to 180°C, more preferably 90 to 160°C, and the duration of holding under heating is usually 10 minutes to 48 hours, preferably 30 minutes to 36 hours, more preferably 1 hour to 24 hours. If the cerium solution is not sufficiently held under heating, the crystallinity of the precipitate to be discussed later may not be improved, resulting in insufficient heat resistance of the objective ceric oxide. Even if the cerium solution is held under heating for a longer time, the heat resistance may be affected little,

and thus being industrially disadvantageous.

In the method of the present invention, following step (b), the heated cerium solution is usually cooled in step (c).

In step (c), the cerium solution may usually be cooled under stirring. Means for cooling are not critical, and may be cooling in an atmosphere or forced cooling with cooling tube. The cooling temperature is usually not higher than 60°C, preferably not higher than 25°C. Through this cooling step (c), a precursor solution is prepared.

In the method of the present invention, a precipitant is added to the cooled cerium solution to prepare a precipitate in step (d). The precipitant used in step (d) may be a base such as sodium hydroxide, potassium hydroxide, an aqueous ammonia solution, ammonia gas, or mixtures thereof, with an aqueous ammonia solution being preferred.

The precipitant may be added by preparing an aqueous solution of the precipitant at a suitable concentration and adding the solution to the precursor solution prepared in step (c) under stirring, or when ammonia gas is used, by blowing the gas into the reaction vessel under stirring. The amount of the precipitant may easily be decided by tracing the pH change of the solution. Usually, a sufficient amount is such that the pH of the solution is not lower than 7, and a preferred amount is such that the pH is 7 to 10.

The precipitation may also be possible by introducing the cooled cerium solution to a precipitant. In this case, required amount of the precipitant is calculated and prepared in advance so that the pH after precipitation may be able to be not lower than 7.

Through the precipitation reaction in step (d), a product with grown crystals may be precipitated. This product is a preferable precursor for obtaining the ceric oxide of the present invention, and may be separated, for example, by Nutsche method, centrifuging, or filter pressing. The precipitate may optionally be washed with water, as required. Further, the precipitate may optionally be dried to a suitable extent for improving the efficiency in the following step (e). In order to further improve the heat resistance of the objective ceric oxide, the precipitate obtained in step (d) may be subjected to, before step (e), step (d-1) of dispersing the precipitate in a solvent such as water, and heat-treating the resulting solution at usually 60 to 220°C, preferably 80 to 180°C, more preferably 90 to 160°C, to obtain a reprecipitate. The duration of the heat treatment is usually 10 minutes to 48 hours, preferably 30 minutes to 36 hours.

According to the present method, the precipitate thus obtained is calcined in step (e) to obtain the objective ceric oxide. In step (e), the calcination temperature may suitably be selected from the range of usually 250 to 900°C. The selection of the temperature may be made as desired, depending on the required or guaranteed values of the specific surface area and bulk density. From a practical point of view to prepare a co-catalyst material wherein the specific surface area is important, the calcination temperature may preferably be 250 to 800°C, more preferably 250 to 700°C, most preferably 280 to 600°C. The duration of calcination may suitably be determined depending on the temperature, and may preferably be 1 to 10 hours.

After step (e), the ceric oxide obtained may usually be pulverized. The pulverization may sufficiently be performed in an ordinary pulverizer, such as a hammer mill, to obtain a powder of a desired particle size. The ceric oxide obtained by the present method may be given a desired particle size through the above mentioned pulverization. For use as a co-catalyst in a catalyst for purifying exhaust gas, for example, a preferred average particle size of the ceric oxide is 0.1 to 50 μm.

It is also possible to add at any point between after step (a) and before step (d) of the process a metallic element compound, for example a nitrate, chloride, oxide, hydroxide, carbonate, halide, oxyhalide, oxynitrate, sulfate, phosphate and/or acetate.

Metallic salt may be made of a cationic metal ion, notably chosen in the group consisting of:

- transition metal elements, such as Zr,
- poor metal elements, such as Al,
- rare earth elements, such as La, Pr, Nd and Y, and
- alkaline earth metal elements, such as Sr.

During or after step (d), an organic texturing agent may be added to the suspension.

An organic texturing agent usually refers to an organic compound, such as a surfactant, able to control or modify the mesoporous structure of the cerium oxide. "Mesoporous structure" basically describes a structure which specifically comprises pores with an average diameter comprised between 2 and 50 nm, described by the term "mesopores". Typically, these structures are amorphous or crystalline compounds in which the pores are generally distributed in random fashion, with a very wide pore-size distribution.

The organic texturing agent may be added directly or indirectly. It can be added directly to the suspension or precipitate resulting from the preceding step. It can also be first added in a composition, for instance comprising a solvent of the organic texturing agent, and said composition being then added to the suspension or precipitate as previously obtained.

The amount of organic texturing agent used, expressed as percentage by weight of additive relative to the weight of the cerium in terms of $CeO_2$, is generally between 5 and 100 % and more particularly between 15 and 60 %.

The organic texturing agent may be adsorbed on the surface of secondary particles and primary particles of the precipitates. For instance, the organic texturing agent adsorbed on the primary particles will lead to increase the size of mesopores and pore volume of the precipitate.

Organic texturing agents are preferably chosen in the group consisting of: anionic surfactants, nonionic surfactants, polyethylene glycols, carboxylic acids and their salts, and surfactants of the carboxymethylated fatty alcohol ethoxylate type. With regard to this additive, reference may be made to the teaching of application WO-98/45212 and the surfactants described in this document may be used.

As surfactants of anionic type, mention may be made of ethoxycarboxylates, ethoxylated fatty acids, sarcosinates, phosphate esters, sulfates such as alcohol sulfates, alcohol ether sulfates and sulfated alkanolamide ethoxylates, and sulfonates such as sulfosuccinates, and alkylbenzene or alkylnapthalene sulfonates.

As nonionic surfactants, mention may be made of acetylenic surfactants, alcohol ethoxylates, alkanolamides, amine oxides, ethoxylated alkanolamides, long-chain ethoxylated amines, copolymers of ethylene oxide/propylene oxide, sorbitan derivatives, ethylene glycol, propylene glycol, glycerol, polyglyceryl esters and ethoxylated derivatives thereof, alkylamines, alkylimidazolines, ethoxylated oils and alkylphenol ethoxylates. Mention may in particular be made of the products sold under the brands Igepal®, Dowanol®, Rhodamox® and Alkamide®.

With regard to the carboxylic acids, it is in particular possible to use aliphatic monocarboxylic or dicarboxylic acids and, among these, more particularly saturated acids. Fatty acids and more particularly saturated fatty acids may also be used. Mention may thus in particular be made of formic acid, acetic acid, propionic acid, butyric acid, isobutyric acid, valeric acid, caproic acid, caprylic acid, capric acid, lauric acid, myristic acid and palmitic acid. As dicarboxylic acids, mention may be made of oxalic acid, malonic acid, succinic acid, glutaric acid, adipic acid, pimelic acid, suberic acid, azelaic acid and sebacic acid.

Salts of the carboxylic acids may also be used, in particular the ammonium.

By way of example, mention may be made more particularly of lauric acid and ammonium laurate.

Finally, it is possible to use a surfactant which is selected from those of the carboxymethylated fatty alcohol ethoxylate type.

The expression "product of the carboxymethylated fatty alcohol ethoxylate type" is intended to mean products consisting of ethoxylated or propoxylated fatty alcohols comprising a $CH_2$-COOH group at the end of the chain.

These products may correspond to the formula :

$$R_1\text{-O-}(CR_2R_3\text{-}CR_4R_5\text{-O})_n\text{-}CH_2\text{-COOH}$$

in which $R_1$ denotes a saturated or unsaturated carbon-based chain of which the length is generally at most 22 carbon atoms, preferably at least 12 carbon atoms; $R_2$, $R_3$, $R_4$ and $R_5$ may be identical and may represent hydrogen or else $R_2$ may represent an alkyl group such as a $CH_3$ group and $R_3$, $R_4$ and $R_5$ represent hydrogen; n is a non-zero integer that may be up to 50 and more particularly between 5 and 15, these values being included. It will be noted that a surfactant may consist of a mixture of products of the formula above for which $R_1$ may be saturated or unsaturated, respectively, or alternatively products comprising both $-CH_2-CH_2-O-$ and $-C(CH_3)-CH_2-O-$ groups.

The alcohol to be used in the method according to the present invention may comprise one or several hydroxyl functions, such as for instance a compound comprising one hydroxyl function, such as ethanol, or a compound comprising two hydroxyl functions, such as ethylene glycol.

The alcohol according to the invention may be a compound of formula (I) :

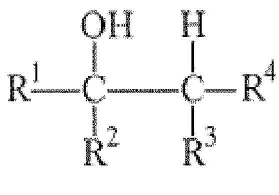

(I)

wherein $R^1$, $R^2$, $R^3$ and $R_4$ are each independently selected from hydrogen or $C_{1-11}$ hydrocarbyl.

Said hydrocarbyl is preferably selected from alkyl, alkenyl, alkynyl, cycloalkyl, cycloalkenyl, or aryl, more preferably selected from alkyl, alkenyl, alkynyl, or aryl, and even more preferably selected from alkyl, alkenyl, or aryl.

Said alkyl preferably comprises 1 to 11 carbon atoms, more preferably 1 to 8 carbon atoms, more preferably 1 to 6 carbon atoms, and even more preferably 1 to 4 (i.e. 1, 2, 3 or 4) carbon atoms. Said alkenyl preferably comprises 2 to 11 carbon atoms, more preferably 2 to 8 carbon atoms, more preferably 2 to 6 carbon atoms, and even more preferably 2 to 4 (i.e. 2, 3 or 4) carbon atoms. Said alkynyl preferably comprises 2 to 11 carbon atoms, more preferably 2 to 8 carbon atoms, more preferably 2 to 6 carbon atoms, and even more preferably 2 to 4 (i.e. 2, 3 or 4) carbon atoms.

Said cycloalkyl preferably comprises 3 to 11 carbon atoms, more preferably 3 to 8 carbon atoms, and even more preferably 3 to 6 (i.e. 3, 4, 5 or 6) carbon atoms. Said aryl is preferably selected from phenyl or naphthyl, and is more preferably phenyl.

Accordingly, said $C_{1-11}$ hydrocarbyl is preferably selected from $C_{1-11}$ alkyl (in particular, $C_{1-6}$ alkyl or $C_{1-4}$ alkyl), $C_{2-11}$ alkenyl (in particular, $C_{2-6}$ alkenyl or $C_{2-4}$ alkenyl), $C_{2-11}$ alkynyl (in particular, $C_{2-6}$ alkynyl or $C_{2-4}$ alkynyl), $C_{3-11}$ cycloalkyl (in particular, $C_{3-6}$ cycloalkyl), $C_{3-11}$ cycloalkenyl (in particular, $C_{3-6}$ cycloalkenyl), phenyl or naphthyl. More preferably, said $C_{1-11}$ hydrocarbyl is selected from $C_{1-11}$ alkyl (in particular, $C_{1-6}$ alkyl or $C_{1-4}$ alkyl), $C_{2-11}$ alkenyl (in particular, $C_{2-6}$ alkenyl or $C_{2-4}$ alkenyl) or phenyl.

In a preferred embodiment, $R^1$, $R^2$, $R^3$ and $R^4$ are each independently selected from hydrogen, $C_{1-11}$ alkyl, $C_{2-11}$ alkenyl, $C_{2-11}$ alkynyl, $C_{3-11}$ cycloalkyl, $C_{3-11}$ cycloalkenyl, phenyl or naphthyl, and are more preferably each independently selected from hydrogen, $C_{1-6}$ alkyl, $C_{2-6}$ alkenyl or phenyl, and are even more preferably each independently selected from hydrogen or linear $C_{1-4}$ alkyl.

In general, it is preferred that the compound of formula (I) comprises a total of 2 to 30 carbon atoms, more preferably 2 to 20 carbon atoms, more preferably 2 to 12 carbon atoms, more preferably 2 to 8 carbon atoms, more preferably 2 to 6 carbon atoms, even more preferably 2 to 5 carbon atoms, and most preferably 2, 3 or 4 carbon atoms.

Moreover, while primary, secondary and tertiary alcohols can generally be used in the method of the invention, it is preferred to use a primary alcohol. Accordingly, it is preferred that one of $R^1$ and $R^2$ comprised in the compound of formula (I) are hydrogen.

It is further preferred that the alcohol to be used in the method according to the invention is a linear (i.e. unbranched) alcohol.

It is particularly preferred that the alcohol (or, accordingly, the compound of formula (I)) is selected from: ethanol; propanol, including propan-1-ol (i.e. n-propanol) and propan-2-ol (i.e. isopropanol); butanol, including butan-1-ol (i.e. n-butanol), 2-methylpropan-1-ol (i.e. isobutanol), 2-methylpropan-2-ol (i.e. tert-butanol) and butan-2-ol (i.e. sec-butanol); or pentanol, including pentan-1-ol (i.e. n-pentanol or amyl alcohol), 3-methylbutan-1-ol (i.e. isopentanol or isoamyl alcohol), pentan-2-ol (i.e. sec-pentanol), pentan-3-ol, 2-methylbutan-1-ol, 3-methylbutan-2-ol (i.e. 1,2-dimethylpropanol) and 2-methylbutan-2-ol (i.e. 1,1-dimethylpropanol).

In one embodiment of the invention, a high purity of alcohol may be preferred. The content of the water in the alcohol may be at most 10 vol. %, preferably at most 5 vol. %, more preferably at most 1 vol. %. Notably, the content of water in the alcohol may be comprised between 0.1 and 10 vol. %, preferably between 0.1 and 5 vol. %, more preferably between 0.1 and 1 vol. %.

With respect to the product of the method according to the invention, the term "alkene" refers to a compound comprising at least one carbon-to-carbon double bond and, in particular, refers to a compound of formula (II) :

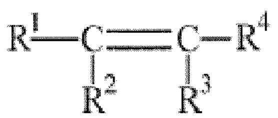

(II)

wherein $R^1$, $R^2$, $R^3$ and $R^4$ have the same meanings and preferred meanings as described and defined herein above for the compound of formula (I).

It is to be understood that all stereoisomers of the compound of formula (I) can be used in the method according to the invention, either in admixture or in pure or substantially pure form. Furthermore, the compound of formula (II) encompasses both cis-isomers and trans-isomers.

In one particularly preferred embodiment the alcohol is ethanol and the produced alkene is ethene (i.e. ethylene). In another particularly preferred embodiment the alcohol is propan-2-ol (i.e. isopropanol) and the produced alkene is propene (i.e. propylene). Yet, in a further particularly preferred embodiment the alcohol is butanol and the produced alkene is butene (i.e. butylene). In this case butanol may be any of n-butanol, tert-butanol, sec-butanol or isobutanol. The produced alkene is either but-1-ene (i.e. $\alpha$-butylene), 2-methylpropene (i.e. isobutylene), or both but-1-ene (i.e. $\alpha$-butylene) and but-2-ene (i.e. $\beta$-butylene).

The method for producing an alkene comprising a step of converting an alcohol by a dehydration step of the present invention usually comprises at least the following steps :

- providing at least an alcohol;
- contacting the alcohol with the catalyst defined as above; thereby producing the alkenes.

The method of producing alkenes by dehydration of an alcohol is notably described in "Ethylene formation by catalytic dehydration of ethanol with industrial considerations", Materials 2013, 6, 101-115, 2012.

Preferably the mixture of alcohol and catalyst is reacted at a temperature range usually comprised between 50 and 500°C, preferably between 300 and 450°C.

Preferably, the method of producing alkenes by dehydration of an alcohol occurs in a single step. In a single step according to the invention means that no intermediate products have to be isolated or that several components have to be produced in separate process steps. Thus, the present invention can be considered as "one pot" procedure, i.e. the catalyst and the alcohols are mixed and brought into contact with each other so that the reaction for producing alkenes can be started.

The dehydration reaction may preferably be conducted in a non-oxidizing atmosphere. As used herein "non-oxidizing atmosphere" means any atmosphere that excludes oxygen and does not lead to the formation of undesirable side reaction products such as $CO_2$. Suitable non-oxidizing atmospheres which can be provided are, for example, inert gases such as $N_2$, He, Ne or Ar.

The dehydration reaction is preferably carried out in a dried atmosphere, meaning an atmosphere comprising between 0.1 and 5 vol. % of water in a liquid or gaseous phase.

The dehydration reaction could also be conducted in the liquid phase, depending on the alcohol and other reaction conditions.

The pressure of the dehydration reaction is not particularly limited, and suitable pressures would be understood to and can be determined by those of ordinary skill in the art. In one embodiment, the dehydration reaction may be, but is not limited to, conducted at a total pressure of about 1 bar to about 20 bar, such as about 5 bar to about 20 bar or 10 bar to about 20 bar or about atmospheric pressure.

The reactor type which may be used in the present invention is not particularly limited, and suitable reactor types would be understood to and can be determined by those of ordinary skill in the art. The reactor may have a simple design or may be complex. The type of reactor may be chosen by the skilled person depending on the components used in the process and the products which should be specifically obtained. In one embodiment, the dehydration reaction may be, but not limited to, conducted in a flow reactor or in a fluid or fluidized bed reactor. The flow reactor is not particularly limited, and suitable flow reactors would be understood to and can be determined by those of ordinary skill in the art. In one embodiment, the flow reactor may be a fixed bed reactor, a up flow fixed bed reactor, or a down flow fixed bed reactor. The fixed bed reactor may be isothermal and/or adiabatic.

Reaction may be carried out in a batch type reactor or in a continuous reaction.

It is notably possible to carry out a pre-oxidation step of the cerium oxide particles under $O_2$, notably to decompose physically adsorbed impurity, such as $CO_2$ or $H_2O$.

At the end of the reaction the alkene as produced may be isolated and purified with known methods, such as for instance solvent extraction, distillation, pressure swing adsorption (PSA) or separation film method.

The following examples are included to illustrate embodiments of the invention. Needless to say, the invention is not limited to described examples.

## EXPERIMENTAL PART

### Example 1

20 g of a ceric nitrate solution in terms of $CeO_2$ containing not less than 90 mol % tetravalent cerium ions was measured out, and adjusted to a total amount of 1 L with deionized water. The obtained solution was heated to 100°C, maintained at this temperature for 24 hours, and allowed to cool down to a temperature of 25°C. Then aqueous ammonium was added to neutralize to pH 8 to obtain cerium oxide hydrate in the form of slurry. The slurry was then subjected to solid-liquid separation with a Nutsche filter to obtain a filter cake. The cake was calcined in the air at 400°C for 10 hours to obtain cerium oxide powder.

The obtained composite oxide powder was measured of the specific surface area by the BET method and of the weight loss by the TG method. Results were reported in Table 1.

### Comparative example 1

200 g of a cerous nitrate solution in terms of $CeO_2$ was introduced into aqueous ammonium which contains 4.18 mol of $NH_4OH$ to obtain cerium hydroxide in the form of slurry. The slurry was then subjected to solid-liquid separation with a Nutsche filter to obtain a filter cake. The cake was calcined in the air at 400°C for 10 hours to obtain cerium oxide powder.

The obtained composite oxide powder was measured in the same way as in Example 1.

**Comparative example 2**

This comparative example relates to a cerium oxide (JRC-CEO-1, Santoku Co).
The composite oxide powder after calcination at 400°C for 10 hours in the air was measured in the same way as in Example 1.

**Comparative example 3**

A cerium oxide powder was prepared in the same way as in Example 1 except that 8.2 g of an ammonium bicarbonate was added after neutralization.
The composite oxide powder was measured in the same way as in Example 1.

**Table 1**

| Compositions | SBET 400°C/10 h ($m^2/g$) | Weight loss at 400°C (%) |
|---|---|---|
| **Invention** 1 | 139 | -0.46 |
| **Comparison 1** | 55 | -0.30 |
| **Comparison 2** | 149 | +2.20 |
| **Comparison 3** | 222 | +3.67 |

The specific surface area is measured by BET method. Use is made of a MOUNTECH Co., LTD. Macsorb analyzer with a 100 mg sample.
The weight loss is measured by TG analysis in the following way. Use is made of a RIGAKU Co., LTD. Thermo plus TG8120 with a 10 mg sample. The sample is heated from ambient temperature to 1000°C in the air with heating rate of 10°C/min. The weight loss of the samples is calculated by the following calculation.

$$\text{Weight loss (\%)} = (A-B)/A*100$$

A : weight of the sample at 350°C detected by TG
B : weight of the sample at 1000°C detected by TG

**Ethanol dehydration activity test**

Ethanol dehydration activity of the sample was conducted by using a fixed-bed flow type reactor under atmosphere pressure. A 0.15 g of the cerium oxide as obtained in previous examples was put in the reactor and subjected to pre-oxidation at 400°C for 1h under $O_2$ gas flow. After the pre-oxidation the $O_2$ gas was replaced to He gas and an ethanol (Wako pure chemicals industries, high purity grade) was introduced in the flow rate of 1.7 ml/h.
The ethanol conversion and the ethylene yield after 0.25 and 4.5 h of the introduction time of the ethanol were analyzed by gas chromatography connected to a flame ionization detector. Results with different compositions were expressed in Table 2.

**Table 2**

| Compositions | Ethanol conversion at 0.25 h (%) | Ethanol conversion at 4.5 h (%) | Ethylene yield at 0.25 h(%) | Ethylene yield at 4.5 h (%) |
|---|---|---|---|---|
| **Invention 1** | 29.3 | 24.6 | 91.3 | 89.1 |
| **Comparison 1** | 10.6 | 7.8 | 85.0 | 73.8 |
| **Comparison 2** | 20.4 | 17.6 | 84.3 | 81.6 |
| **Comparison 3** | 11.3 | 7.5 | 83.2 | 74.2 |
| **Comparison 4 (Blank)** | 2.1 | nm | 0 | nm |
| nm: non measured | | | | |

It appears then that the catalyst of the present invention allows a dehydration of ethanol with higher conversion and yield in comparison with the catalysts of the prior art having lower specific surface area or purity as represented by the weight loss parameter.

## Claims

**1.** A method for producing an alkene comprising a step of converting an alcohol by a dehydration step into an alkene with cerium oxide particles as catalyst, wherein said cerium oxide particles have the following properties :

- a specific surface area comprised between 100 and 300 $m^2/g$, after calcination at 400°C for 10 hours; and
- a weight loss comprised between -1.0 and +1.0 %, between a temperature of 350°C and 1000°C, as measured by a Thermal Gravimetric Analysis.

**2.** The method according to claim 2 wherein cerium oxide particles have a specific surface area comprised between 30 and 65 $m^2/g$, after calcination at 900°C for 5 hours.

**3.** The method according to claim 1 or 2 wherein cerium oxide particles have a total pore volume comprised between 0.7 and 1.5 ml/g after calcination at 900°C for 5 hours, under air.

**4.** The method according to anyone of claims 1 to 3 wherein cerium oxide particles have a S1/S2 ratio comprised between 0.45 and 0.7 taken after calcination at 800°C for 2 hours.

**5.** The method according to anyone of claims 1 to 4 wherein cerium oxide particles comprise at least one metallic element oxide, other than cerium oxide.

**6.** The method according to anyone of claims 1 to 5 wherein cerium oxide particles have a weight loss comprised between -0.6 and +0.5 %, between a temperature of 350°C and 1000°C, as measured by a Thermal Gravimetric Analysis.

**7.** The method according to anyone of claims 1 to 6 wherein cerium oxide particles are obtained by a method comprising at least the steps of:

(a) providing a cerium solution not less than 90 mol % of which cerium ions are tetravalent,
(b) holding said cerium solution prepared in step (a) at a temperature from 60 to 220°C under heating,
(c) cooling said heated cerium solution,
(d) adding a precipitant to said cooled cerium solution to obtain a precipitate, and
(e) calcining said precipitate.

**8.** The method according to anyone of claims 1 to 7 wherein the alcohol is a compound of formula (I) :

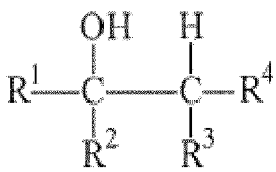

(I)

wherein $R^1$, $R^2$, $R^3$ and $R^4$ are each independently selected from hydrogen or $C_{1-11}$ hydrocarbyl.

**9.** Method according to claim 8 wherein $C_{1-11}$ hydrocarbyl is selected in the group consisting of: alkyl, alkenyl, alkynyl, cycloalkyl, cycloalkenyl, and aryl.

**10.** The method according to anyone of claims 1 to 9 wherein the alcohol is selected in the group consisting of: ethanol, propanol, butanol, and pentanol.

**11.** The method according to anyone of claims 1 to 10 wherein the content of water in the alcohol is comprised between

0.1 and 10 vol. %.

**12.** The method according to anyone of claims 1 to 11 wherein the alkene is a compound of formula (II) :

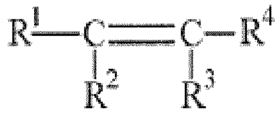

(II)

wherein $R^1$, $R^2$, $R^3$ and $R^4$ are each independently selected from hydrogen or $C_{1-11}$ hydrocarbyl.

**13.** The method according to anyone of claims 1 to 12 wherein then method for producing an alkene comprises at least the following steps :

- providing at least an alcohol;
- contacting the alcohol with the catalyst defined as above; thereby producing the alkenes.

**14.** The method according to anyone of claims 1 to 13 wherein the mixture of alcohol and cerium oxide particles is reacted at a temperature comprised between 50 and 500°C.

**15.** The method according to anyone of claims 1 to 14 wherein the method of producing an alkene occurs in a single step.

**16.** The method according to anyone of claims 1 to 15 wherein the reaction is carried out in a non-oxidizing atmosphere.

Europäisches Patentamt
European Patent Office
Office européen des brevets

# EUROPEAN SEARCH REPORT

Application Number
EP 15 16 0076

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2014/103898 A1 (KAO CORP [JP]) 3 July 2014 (2014-07-03) * examples 1, comparative 1 * ----- | 1-6,8-16 | INV. C07C1/24 C07C11/04 |
| A | US 5 210 363 A (SWEENEY WILLIAM A [US]) 11 May 1993 (1993-05-11) * examples 4b,8 * ----- | 1-16 | |
| A | US 4 873 392 A (LE VAN MAO RAYMOND [CA]) 10 October 1989 (1989-10-10) * the whole document * ----- | 1-16 | |
| A | US 4 490 567 A (DRAKE CHARLES A [US]) 25 December 1984 (1984-12-25) * the whole document * ----- | 1-16 | |

TECHNICAL FIELDS SEARCHED (IPC)

C07C

The present search report has been drawn up for all claims

1

| Place of search | Date of completion of the search | Examiner |
|---|---|---|
| The Hague | 18 September 2015 | Megido, Benigno |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 15 16 0076

This annex lists the patent family members relating to the patent documents cited in the above-mentioned European search report. The members are as contained in the European Patent Office EDP file on
The European Patent Office is in no way liable for these particulars which are merely given for the purpose of information.

18-09-2015

| Patent document cited in search report | Publication date | Patent family member(s) | Publication date |
|---|---|---|---|
| WO 2014103898 A1 | 03-07-2014 | CN 104884412 A | 02-09-2015 |
| | | DE 112013006222 T5 | 24-09-2015 |
| | | JP 2014141466 A | 07-08-2014 |
| | | WO 2014103898 A1 | 03-07-2014 |
| US 5210363 A | 11-05-1993 | NONE | |
| US 4873392 A | 10-10-1989 | NONE | |
| US 4490567 A | 25-12-1984 | CA 1189848 A1 | 02-07-1985 |
| | | DE 3377268 D1 | 11-08-1988 |
| | | EP 0091662 A2 | 19-10-1983 |
| | | US 4490567 A | 25-12-1984 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7361322 B
- WO 9845212 A


### Non-patent literature cited in the description


- *The Journal of the American Chemical Society,* 1938, vol. 60, 309
- Ethylene formation by catalytic dehydration of ethanol with industrial considerations. *Materials 2013,* 2012, vol. 6, 101-115